# EUROPEAN PATENT APPLICATION

(11) **EP 4 033 864 A1**
(43) Date of publication of application: **27.07.2022**
(21) Application number: 20866010.0
(22) Date of filing: 18.09.2020
(51) Int. Cl.: H05B 45/20, H05B 47/16, A61N 5/06

(54) **SELF-CONTAINED LIGHTING DEVICE FOR CIRCADIAN RHYTHM REGULATION**

(30) Priority: 20.09.2019 ES 201931533 U
(71) Applicant: Normagrup Technology, S.A., 33428 Llanera (Asturias) (ES)
(72) Inventor: González Soriano, Aurelio, 33428 LLANERA (Asturias) (ES)
(74) Representative: De Castro Hermida, José Luis
(86) International application number: PCT/ES2020/070562
(87) International publication number: WO 2021/053258

(57) **Abstract**

The invention relates to a self-contained lighting device for circadian rhythm regulation, which does not require a connection to any external element or device for its operation, and has the particularity of comprising: at least one LED lamp that emits light into the environment; an adapter/transformer of the LED lamp that controls the colour of the light, and is connected to a microcontroller; a microcontroller which is an electronic module with flash memory and which also comprises a real-time clock; a power supply, connected to the microcontroller; at least one crystal oscillator, connected to the microcontroller; and which may comprise an energy-saving light sensor and may comprise a button for adjusting the device.

## Description

### OBJECT OF THE INVENTION

The invention consists of a lighting device that allows the creation of healthy, comfortable, and efficient lighting environments, and specifically allows the simulation of circadian cycles without the need for external communication elements that regulate said operation.

The invention falls within the different pieces of equipment and devices related to lighting, and more specifically addresses the types of luminaires that are self-contained and adjustable.

### BACKGROUND OF THE INVENTION

The circadian cycle is a technical concept that refers to the biological clock that regulates and programs the physiological functions of an organism in a period of one day or 24 hours and it is known, within this technical field, that the circadian cycles of living organisms are generally associated with periods of light and darkness.

In this sense, it is known that the changes in lifestyle and time schedules that humans have today, together with the lack of exposure to natural light and the excess of artificial light, alter our biological rhythms and, therefore, affect our health and performance. These conditions are a problem that goes beyond the psychological field, but directly affects the health of the population in general.

There are solutions designed to improve this problem, such as that disclosed in document ES2259885, where a nutritional kit is described that comprises a wake-promoting formula and a sleep-promoting formula and which is preferably intended for children.

In this regard, what is disclosed in document ES8604142 is known, where a procedure for obtaining melatonin that is used to regulate the circadian cycle is described.

More related to the technical field of the present invention, what is disclosed in document WO2017054784 is known, where a method is described that uses lamps that emit monochromatic or coloured light and that display texts, graphics and moving images, with which generate interaction mechanisms and narrative models that are used to improve anxiety control management, sleep cycle management, habit change, the ability to improve ways of feeling the environment, and in general for the improvement in the relationship with other living beings.

Taking into account the known background within this technical field, it is considered that there is no lighting device that can be used in a simple way for the regulation and simulation of circadian cycles, and where messages or additional elements are not emitted, it simply regulates the colour/temperature of light. The present invention consists of a self-contained luminaire that can be installed by any user at home or in any other more professional environment such as offices, educational centres, hospitals; it is a luminaire that is connected like any standard luminaire to the power grid; and it is a luminaire that does not require connection to any centralized communications system, therefore, it is not necessary to carry out any programming so that the equipment begins to function according to the circadian cycle.

In this sense, it must be taken into account that the retina of the human eye has two types of photoreceptors, the rods for night and peripheral vision, and the cones that are responsible for colour vision. These photoreceptors are responsible for sending information to the visual processing centres of the brain through the ganglion cells and for managing everything related to visual aspects. Some of the ganglion cells are intrinsically photosensitive and also act as photoreceptors. In this case, the treatment of light through these cells regulates non-visual aspects of human beings that regulate the biological clock, the production of some hormones, sleep, or others. These ganglion cells are preferably stimulated by wavelengths between 460 and 490nm.

It is known that light rich in blue content stimulates the ganglion cells that control the production of dopamine that plays an important role in muscle coordination, alertness, and pleasure; serotonin that works as a stimulant and motivator and therefore helps to raise energy levels; and cortisol (stress hormone) that stimulates metabolism and programs the body for the day. At the same time, the same light rich in blue content suppresses the production of melatonin, which is the hormone that makes us feel tired, slows down the body's functions and reduces activity to promote rest and reduce fatigue.

Therefore, taking into account both the aforementioned problems and the known solutions, also taking into account the knowledge of the effects of light on a user, it is considered that the present invention improves the conditions given that, unlike any known technique, manages to create healthy, comfortable, and efficient lighting environments by simply regulating the intensity of the light emitted by said luminaire according to the time and date in which the device is used.

### DESCRIPTION OF THE INVENTION

As previously mentioned, there are currently many health problems derived from the current lifestyle and its time schedules, the lack of exposure to natural light and the excess of artificial light.

The luminaire object of the present invention is a self-contained device that allows creating light environments that simulate or regulate circadian cycles.

The luminaire object of the present invention is a self-contained equipment that incorporates an internal clock that automatically maintains the date, time, and season of the year. These data are stored in such a way that operation is guaranteed, and once the equipment is installed and connected to the power grid, it begins to reproduce the circadian cycle autonomously.

As previously stated, the present device does not require a connection to any centralized communications system and no programming is required for the equipment to start operating according to the circadian cycle. One of the peculiarities and advantages is that the device simply connects like any standard luminaire to the power grid. Despite not having a centralized communications system, it is possible to synchronize the time of the luminaires.

Going into the detail of the invention, the device consists of:
- at least one LED lamp, which emits light into the environment;
- an LED adapter/transformer, known as a "driver;" in charge of controlling the colour of the light, preferably with a variation of colour from the coldest to the warmest temperatures, and which is connected to and controlled by a microcontroller;
- a microcontroller, which is an electronic module with a flash memory, and which further comprises a real-time clock;
- a power supply, which can be a battery and/or wiring for connection to the conventional power grid, connected to the microcontroller;
- at least one 32768 Hz crystal oscillator, connected to the microcontroller, which stabilizes the operation of the clock.

The microcontroller, which incorporates a real-time clock, in its memory comprises two modules for circadian cycles with different behaviour:
- a fixed cycle module with predetermined start and finish times and a duration of 12 hours; and
- a real cycle module with start and finish coinciding with the sunrise and sunset times.

This real-time clock can incorporate algorithms to adapt the circadian cycle to 5 different latitudes and for the northern and southern hemispheres, which allows, also taking into account the time of the adjustable luminaire, to be able to adapt the operation depending on the location of the device.

In an embodiment of the invention, additionally to the electrical power supply, which as indicated may be a battery and/or wiring for connection to the conventional power grid and which is connected to the microcontroller, the invention may comprise a backup power supply so that the real-time clock can continue operating even if the main power supply fails.

In one embodiment of the invention, the device comprises at least one light sensor, connected to the microcontroller, which is a sensor that continuously measures and detects the amount of light in the environment, so that the light from the luminaire is adapted by the microcontroller, reducing, and adjusting the power, and saving energy.

In an embodiment of the invention, the LED lamp comprises a plurality of diodes. On the other hand, the electrical power supply (5) can be a lithium battery.

To improve and clarify the explanation, it is understood that LED lights emit at certain colour temperatures, but to unify the description from now on, we speak of LED colour, and it is also clarified that in general in this industrial field it is understood that there are 3 main colours of LED light: warm light (up to around 3300K), neutral light (around between 3300-5000K) and cool light (around between 5000-6500K).

For this, the microcontroller, in a particular embodiment, can comprise a programmable module comprising a programming or algorithm that calculates the spectrum of light at all times so that it approaches that of the sun, and distributes the power of the lamp between the diodes of cold, warm, and neutral LEDs. The high resolution of the microcontroller and the pulse width modulation make the transitions between the different light spectra and levels smooth.

As an example of the behaviour of a fixed cycle, the device performs a 12-hour circadian cycle, especially designed for work centres. The start of the cycle starts at 06:00 and ends at 18:00. During the cycle, the colour temperature varies between 2700K and 5600K. In the following Table 1 a matrix can be seen that is entered in the memory of the microcontroller and with the relationship between said hours and the colour of light emission.

**Table 1**

| Time | Colour |
|---|---|
| 6:00 | 2700K |
| 7:00 | 3500K |
| 8:00 | 4300K |
| 9:00 | 4800K |
| 10:00 | 5100K |
| 11:00 | 5400K |
| 12:00 | 5600K |
| 13:00 | 5400K |
| 14:00 | 5100K |
| 15:00 | 4800K |
| 16:00 | 4300K |
| 17:00 | 3500K |
| 18:00 | 2700K |

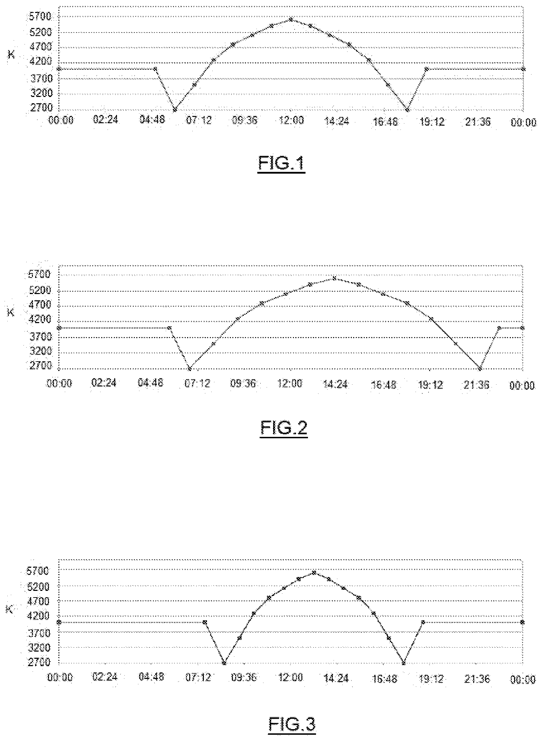

In this way, and according to what can be seen in Fig.1, the device gradually emits light at said temperature autonomously during this period.

In an example of the behaviour of a real cycle, the device performs a cycle where the official sunrise and sunset time data are taken. In the case of Spain, the data published by the National Geographic Institute are taken into account. In this sense, it is possible to download the sunrise and sunset times of all the provinces. Here is the problem that between the most distant provinces there is a time difference of close to one hour. As an example, between the cities of A Coruña and Palma de Mallorca there is precisely one hour of time difference. To solve this problem, at least in Spain, given that the time of sunset and sunrise depends on the geographical longitude of the place, the values of the city of Madrid are taken, since it is located from a geographical point of view in the centre of the country, so that for any user who is located in another province, the error regarding the exact time of sunrise and sunset will be small. Therefore, the following table 2, which can be downloaded from the website of the National Geographic Institute, is entered into the memory of the microcontroller in the form of a matrix in its flash memory. In this example, the cycle corresponds to latitude 40° of the northern hemisphere (which corresponds to Spain) and taking the exact data of the sunrise and sunset times of the geographical point and eliminating the time changes. As indicated, the sunrise and sunset data for all latitudes are entered in the form of a matrix in the memory of the microcontroller.

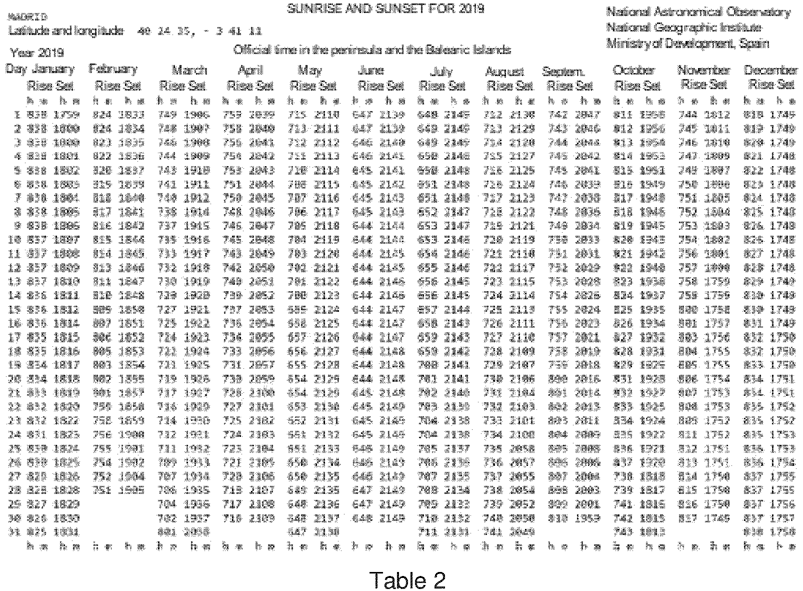

The matrix shown in table 2 is the exact hours and minutes in which sunrise and sunset occur on each and every day of the year in Madrid. Source National Geographic Institute.

In this way, the device, with its real-time clock, can know exactly the sunrise and sunset times for every day of the year. Based on sunrise and sunset times, the cycle or "fixed cycle" that we have seen previously is adapted, and instead of taking 06:00 and 18:00, the real values of sunrise and sunset for that geographical position will be taken, the duration of the cycle being variable instead of fixed, with a maximum duration at the Summer Solstice (June 21) and a minimum duration at the Winter Solstice (December 21).

In the following Table 3 the matrix recalculated according to the previous parameters with the relationship between the times and the colour of light emission for the Summer Solstice date can be seen.

**Table 3**

| Time | Colour |
|---|---|
| 6:45 | 2700K |
| 8:00 | 3500K |
| 9:15 | 4300K |
| 10:30 | 4800K |
| 11:46 | 5100K |
| 13:01 | 5400K |
| 14:16 | 5600K |
| 15:31 | 5400K |
| 16:47 | 5100K |
| 18:02 | 4800K |
| 19:17 | 4300K |
| 20:32 | 3500K |
| 21:48 | 2700K |

In this way, and according to what can be seen in Fig.2, the device during that period on that specific date gradually emits light at said temperature autonomously.

On the other hand, in Table 4 the matrix recalculated according to the previous parameters with the relationship between the times and the colour of light emission for the Winter Solstice date can be seen.

**Table 4**

| Time | Colour |
|---|---|
| | |
| 8:34 | 2700K |
| 9:20 | 3500K |
| 10:06 | 4300K |
| 10:53 | 4800K |
| 11:39 | 5100K |
| 12:26 | 5400K |
| 13:12 | 5600K |
| 13:58 | 5400K |
| 14:45 | 5100K |
| 15:31 | 4800K |
| 16:18 | 4300K |
| 17:04 | 3500K |
| 17:51 | 2700K |

In this way, and according to what can be seen in Fig3, the device during that period on that specific day gradually emits light at said temperature autonomously.

Furthermore, the device can be adjustable and programmed, for which the device comprises a programmable module and can comprise at least one push button or button connected to the microcontroller. The present invention is a self-contained device that does not require connection to other electrical devices but to the power grid. Through the connection to the power grid, it can have some adjustment modes through a simple switch connected to the microcontroller, and that allows the synchronization of the clock time in real time, the adjustment of the dimmer gain and the selection among a plurality of latitudes and the two hemispheres. To do this, by using a switch and pressing it, it is possible to cause interruptions in the supply voltage of the device that allow adjustment to the following programmed parameters:
- time synchronization and latitude adjustment, preferably to be selected from latitudes 0°, 20°, 30°, 40° or 50°; with which the device can be adapted to different locations in the world;
- adjustment of the dimmer, preferably to be selected from a low level, medium level, high level, or deactivation; and
- the adjustment of the hemisphere between North and South.

For this adjustment, pre-programmed sequences are applied in a programmable module of the microcontroller consisting of very short pulse or power-on sequences followed by stop or power-off times, preferably in power-on time sequences of 1 second versus power-off times of 5 seconds. seconds. In this adjustment mode, the device emits a sequence of flashes, which vary based on the specifications of the machine, specifically in intensity or colour temperature (warm, neutral, or cold) and in speed (fast, slow, or fixed), where preferably the slow flashing is performed with a frequency of 1 Hz and the fast flashing is performed with a frequency of 4 Hz.

It should be noted that, throughout the description and claims, the term "comprises", and its variants are not intended to exclude other technical characteristics or additional elements.

### BRIEF DESCRIPTION OF THE FIGURES OF THE INVENTION

In order to complete the description and to help a better understanding of the characteristics of the invention, a set of figures and drawings is presented wherein, by way of illustration and not limitation, the following is represented:
Figure 1 is a diagram showing temperature/colour (K) in regard to hours of a day in a fixed circadian cycle.
Figure 2 is a diagram showing temperature/colour (K) in regard to hours of a day in an actual circadian cycle for the Summer Solstice date (June 21).
Figure 3 is a diagram showing temperature/colour (K) in regard to hours of a day in an actual circadian cycle for the Winter Solstice date (December 21).
Figure 4 is a schematic representation of an embodiment of the internal composition of the device object of the present invention that emits light into the environment.
Figure 5 is a schematic representation of an embodiment which also comprises both a light sensor (8) and a push button for adjusting the device.

### DETAILED DESCRIPTION OF EMBODIMENTS OF THE INVENTION

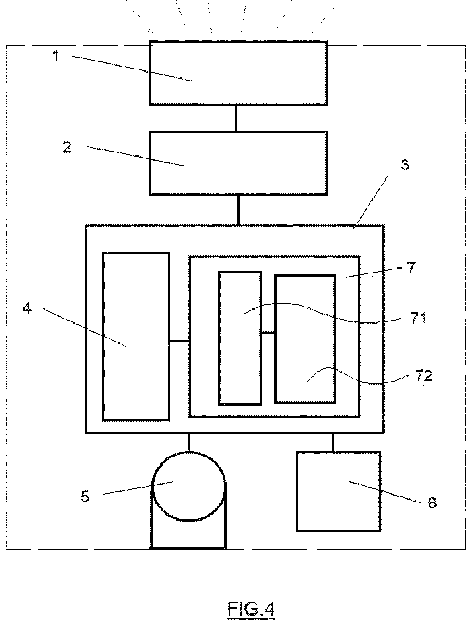

Taking into account the previous figures 1 to 4, it can be seen that, in an embodiment of the invention, the self-contained lighting device for the regulation of circadian cycles internally comprises:
- at least one LED lamp (1), which emits light into the environment;
- an adapter/transformer (2) for the LED lamp, responsible for controlling the colour of the light, preferably with a colour variation between 2700K and 5600K, and which is connected to and controlled by a microcontroller (3);
- a microcontroller (3), which is an electronic module with a flash memory (7), and which further comprises a real-time clock (4);
- an electrical power supply (5), which can be a battery and/or a wiring for connection to the conventional power grid, connected to the microcontroller;
- at least one 32768 Hz crystal oscillator (6), connected to the microcontroller; and
where the microcontroller comprises in its memory some modules for circadian cycles with a different behaviour, specifically:
- a fixed cycle module (71) with predetermined start and finish times and with a duration of 12 hours; and a real cycle module (72) with start and finish coinciding with the sunrise and sunset times.
- or a circadian cycles module with start and finish coinciding with the sunrise and sunset times for different latitudes (at least 5 latitudes) and two hemispheres
and wherein, in addition, the device can include a backup power supply so that the real-time clock continues operating when the main power supply may fail.

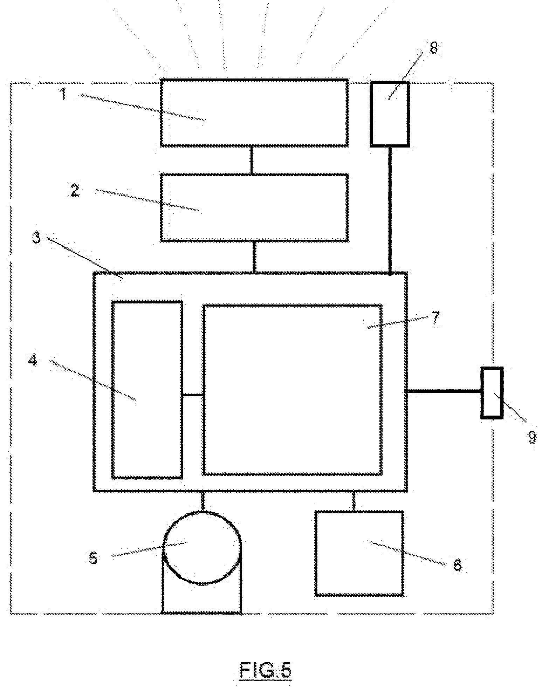

In Figure 5 an embodiment of the device can be seen where, in addition, the device can comprise a light sensor (8) connected to the microcontroller (3) with which the presence of light in the environment can be detected and power consumption can be managed and saved. In this embodiment, moreover, the microcontroller comprises a programmable module, and the device comprises at least one push button (9) with which a user can adjust the microcontroller (3) by means of a pre-programmed sequence of pressing and stopping said push button.

## Claims

1. Self-contained lighting device for the regulation of circadian cycles, **characterized in that** internally it comprises:
- at least one LED lamp (1) that emits light into the environment;
- an adapter/transformer (2) of the LED lamp that controls the colour of the light, and that is connected to a microcontroller;
- a microcontroller (3), which is an electronic module with a flash memory (7), and which further comprises a real-time clock (4);
- an electrical power supply (5), connected to the microcontroller; and
- at least one crystal oscillator (6), connected to the microcontroller.

2. Self-contained lighting device for the regulation of circadian cycles, according to claim 1, wherein the memory (7) of the microcontroller comprises:
- a fixed cycle module (71) with predetermined start and finish times and with a duration of 12 hours; and
- a real cycle module (72) with start and finish coinciding with the sunrise and sunset times.

3. Self-contained lighting device for the regulation of circadian cycles, according to claim 1, wherein the light is emitted with a colour that varies between 2700K and 5600K.

4. Self-contained lighting device for the regulation of circadian cycles, according to claim 1, wherein the crystal oscillator (6) is of 32768 Hz.

5. Self-contained lighting device for the regulation of circadian cycles, according to claim 1, wherein the electrical power supply (5) is a battery or is a wiring for connection to the conventional power grid.

6. Self-contained lighting device for the regulation of circadian cycles, according to claim 1, wherein the memory (7) of the microcontroller comprises a module of circadian cycles with start and finish coinciding with the sunrise and sunset times for different latitudes and the two hemispheres.

7. Self-contained lighting device for the regulation of circadian cycles, according to claim 1, **characterized in that** the device comprises a backup power supply for the real-time clock (4).

8. Self-contained lighting device for the regulation of circadian cycles, according to claim 1, **characterized in that** it comprises at least one light sensor (8) connected to the microcontroller (3).

9. Self-contained lighting device for the regulation of circadian cycles, according to claim 1, wherein the microcontroller (3) comprises a programmable module.

10. Self-contained lighting device for the regulation of circadian cycles, according to claim 9, **characterized in that** it comprises at least one adjustment push button (9) connected to the programmable module of the microcontroller (3).

11. Self-contained lighting device for the regulation of circadian cycles, according to claim 1, wherein the power source (5) is a lithium battery.

12. Self-contained lighting device for the regulation of circadian cycles, according to claim 1, wherein the LED lamp (1) comprises a plurality of diodes.
